# EUROPEAN PATENT APPLICATION

(11) **EP 1 321 163 A1**
(43) Date of publication of application: **25.06.2003**
(21) Application number: 01310633.1
(22) Date of filing: 19.12.2001
(51) Int. Cl.: A61M 25/06, A61M 25/00

(54) **Catheter Assembly**

(71) Applicant: C-I-Medic Co., Ltd., Sendai-shi, Miyagi-ken (JP); ASAHI INTECC CO., LTD., Nagoya-shi, Aichi-ken (JP)
(72) Inventor: Meguro, Taiichiroh, Aoba-ku, Sendai-shi, Miyagi-ken (JP); Kawahara, Yasuyuki, Nagoya-shi, Aichi-ken (JP); Jiang, Lan, Nagoya-shi, Aichi-ken (JP); Momota, Masashi, Nagoya-shi, Aichi-ken (JP); Miyata, Masahiko, Nagoya-shi, Aichi-ken (JP)
(74) Representative: Nicholls, Michael John

(57) **Abstract**

With an introducing catheter (A) accommodated by a diagnostic-therapeutic catheter (B), the necessity of a sheath is eliminated to render the diagnostic-therapeutic catheter (B) as large as the sheath. This enables a manipulator to fully treat a patient with the use of diagnostic-therapeutic catheter (B). With the elimination of the sheath, the diagnostic-therapeutic catheter (B) is directly inserted into tubular human organs (e.g., radial artery and brachial artery). Coaxially provided are inner and outer tubes 12, 13 with the diagnostic-therapeutic catheter (B) to have an annular space 14, at least a part of which forms a drug-releasable open end 15 exposed to a shaft portion 11. The outer tube 13 has a plurality of drug-releasable side holes 9 to release an anti-spasmodic drug toward a blood vessel through the drug-releasable open end 15.

## Description

The invention relates to a catheter assemble of a diagnostic-therapeutic catheter and an introducing catheter which is improved to be directly inserted into a human tubular organs, particularly a fragile blood vessel of an arm wrist or the like.

Upon diagnosing to percutaneously and transvascularly treat human organs and a blood vessel system through a skin and blood vessel, a sheath is inserted into the blood vessel beforehand, and a diagnostic-therapeutic catheter is guided by the sheath to be inserted into the blood vessel.

At the time of treating cardio-ischemic diseases such as angina pectoris and myocardial infarction, percutaneously and transvasucularly conducted PTCA (Percutaneous Transluminal Coronary Angioplasty) is a current trend rather than a coronary bypass surgery to mitigate pains a patient suffers during the operation.

Depending on where the catheter is to be inserted, the PTCA is divided in chief into three categories, i.e., a trans-femoral approach, trans-brachial approach and trans-radial approach.

In the trans-femoral approach, it is necessary to insert a urinary catheter beforehand into a bladder to insure a post-surgery urination, which increasingly inflicts the patient, while at the same time taking a long time to do a post-surgery styptic treatment.

This requires to hospitalize the patient quietly for an extended period of time. Recently, an attention is drawn to a less interventional treatment which lessens the burden the patient suffers to improve a quality of patient's life.

In the trans-radial approach as opposed to the trans-femoral approach, the necessity of urinary catheter is eliminated so that the patient can get out of the bed immediately after treated the patient. This relieves the patient of a longer rest in hospital, and as a result, the patient needs to be hospitalized such a short stay as only one day, thereby significantly mitigating the burdens and pains the patient suffers.

However, owing to the small radial artery, an insertable sheath is substantially constricted to ones having the size around 6 Fr (approx. 2.67 mm in OD) The catheter guided by the sheath is limited to only ones having maximum size around 6 F (approx. 2.00 mm in OD).

When using an 8 Fr sheath (approx. 3.34 mm in OD), patients who have a blood vessel large enough to admit the sheath insertion of this dimension are very limited.

Upon conducting the PTCA, used are a balloon dilatation, stent implant, Rotablator (diamond-coated Burr rotating at up to 190,000 rpm ablates plaque into fine particles), DCA (catheter shaving and removing atherosclerotic material from a stenosed area of the blood vessel in association with a movement of the cutter), kissing balloons (balloons inserted at the same time into the stenosed area are inflated).

When using an 8 F catheter (approx. 2.67 mm in OD), it is possible to fully cure the blood stenosed-related disease by the PTCA. When using a 6 F catheter on the other hand, it is difficult to place the Rotablator Burr (more than 1.5 mm in OD) or the balloons in the catheter, and thereby rendering it hard to fully treat the patient.

The trans-radial approach often sends the blood vessel into a spasm when manipulating the sheath to cut through the blood vessel so as to guide the catheter into the blood vessel. When forcibly withdrawing the catheter and the sheath from the blood vessel, the withdrawing action may inflict greatly on the patient to induce an internal bleeding due to the blood vessel's endothelia being injured, raptured or dissociated.

When using a long sized sheath, a sharp and hard distal end of the long sized sheath may injure a heart and other organs. Upon inserting the long sized sheath into the tubular human organ, the sharp distal tip end of the long sized sheath may locally concentrate on one point of the tubular human organ and the sharp edge may be broken. The broken section of the long sized sheath may injure the human organ or the blood vessel tissues.

Since the long sized sheath generally has a hemostasis valve at a near open rear end, the hemostasis valve hinders collateral remedial tools such as PTCA dilatation catheters to be inserted.

Further, the long sized sheath is not sufficient in torque transmission and pushability, etc., to put into a practical use in combination with the catheter.

Therefore, the present invention has made with the above drawbacks in mind, it is a main object of the invention to provide a catheter assemble of a diagnostic-therapeutic catheter and an introducing catheter which is capable of directly inserting into the human blood vessel system without using a sheath.

It is another object of the invention to provide a catheter assemble of a diagnostic-therapeutic catheter and a introducing catheter which is capable of rapidly releasing drug through drug-releasable side holes and a drug-releasable front open end so as to instantly relieve a patient of a spasm occured on the blood vessel when inserting the diagnostic-therapeutic catheter and the introducing catheter into the blood vessel.

According to the present invention, there is provided a catheter assemble of a diagnostic-therapeutic catheter and an introducing catheter. A distal portion of the introducing catheter has a taper-terminated portion, a linear portion, a linear-terminated portion and a stepped portion, either of which substantially engages tightly with a distal portion of the diagnostic-therapeutic catheter without a gap (space) or clearance. An annular space is provided beteween a shaft portion of the diagnostic-therapeutic catheter and the introducing catheter except for the distal portion of the shaft portion.

Such is the structure that the necessity of a sheath is eliminated. With the elimination of the sheath, it is possible to insert a larger catheter into a human organ. This enables a manipulator to insert an 8 F catheter into substantially every patient's radial artery so as to fully treat the patient. This considerably mitigates pains the patient suffers compared to the prior art counterpart in which the catheter of the same size is used with the sheath.

In order that the invention may be better understood, the following description is given, only by way of example, with reference to the accompanying drawings in which:
Fig. 1 is a plan view of an introducing catheter according to a first embodiment of the invention;
Fig. 2 is a plan view of a diagnostic-therapeutic catheter;
Fig. 3 is a plan view of an assemble of the diagnostic-therapeutic catheter and the introducing catheter;
Fig. 4 is a longitudinal cross sectional view of a distal portion of the introducing catheter;
Fig. 5 is a longitudinal cross sectional view of the diagnostic-therapeutic catheter;
Fig. 6 is an enlarged longitudinal cross sectional view of the diagnostic-therapeutic catheter into which the introducing catheter is inserted;
Fig. 7 is a longitudinal cross sectional view of the assemble inserted into a radial artery;
Fig. 8 is a longitudinal cross sectional view of the diagnostic-therapeutic catheter in which the introducing catheter is withdrawn from the assemble;
Fig. 9 is an enlarged plan view of drug-releasable side holes provided with the diagnostic-therapeutic catheter according to a second embodiment of the invention;
Fig. 10 is a schematic view of a distal portion of the diagnostic-therapeutic catheter having an inner edge rounded according to a third embodiment of the invention;
Fig. 11 is a plan view of the distal portion of the diagnostic-therapeutic catheter having the inner edge rounded;
Fig. 12 is a schematic view showing that the diagnostic-therapeutic catheter comes in surface contact with a blood vessel;
Fig. 13 is a schematic view showing that a prior art catheter comes in point contact with an entry of coronary artery;
Fig. 14 is a schematic view showing that a front open end of the prior art catheter is broken due to a stress concentration;
Fig. 15 is a plan view of an introducing catheter according to a fourth embodiment of the invention;
Fig. 16 is a plan view of a drug-releasable diagnostic-therapeutic catheter;
Fig. 17 is a plan view of a catheter assemble of the diagnostic-therapeutic catheter and the introducing catheter;
Fig. 18 is a longitudinal cross sectional view of a distal portion of the introducing catheter;
Fig. 19 is a longitudinal cross sectional view of a distal portion of the diagnostic-therapeutic catheter;
Fig. 20 is an enlarged longitudinal cross sectional view of the diagnostic-therapeutic catheter into which the introducing catheter is inserted;
Fig. 21 is a longitudinal cross sectional view of the catheter assemble inserted into a radial artery;
Fig. 22 is a longitudinal cross sectional view of the diagnostic-therapeutic catheter in which the introducing catheter is withdrawn from the catheter assemble;
Fig. 23 is a development view showing a relationship between drug-releasable holes of the same size;
Fig. 24 is a development view showing a relationship between the drug-releasable holes of different sizes;
Fig. 25 is a development view showing the drug-releasable holes arranged in row and column; and
Fig. 26 is a latitudinal cross sectional view of grooves formed on an inner tube of a drug-releasable diagnostic-therapeutic catheter.

### DETAILED DESCRIPTION OF PREFERRED EMBODIMENTS OF THE INVENTION

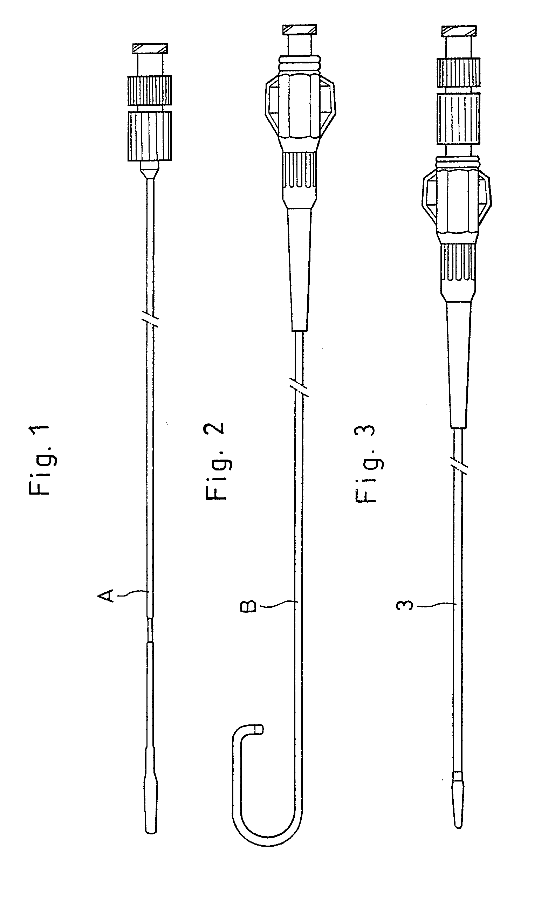

Referring to Figs. 1 through 8 which show a first embodiment of the invention, Figs. 1, 2 and 3 in turn depict an introducing catheter (A), a tubular diagnostic-therapeutic catheter (B) and an assemble of the introducing catheter (A) and the diagnostic-therapeutic catheter (B).

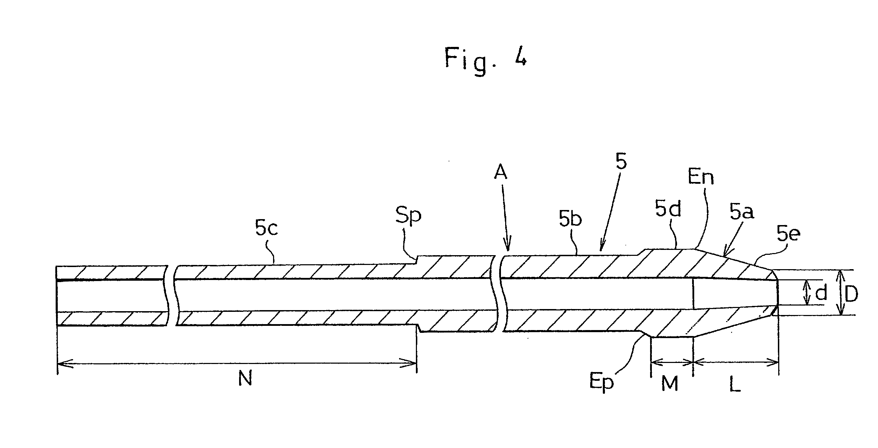

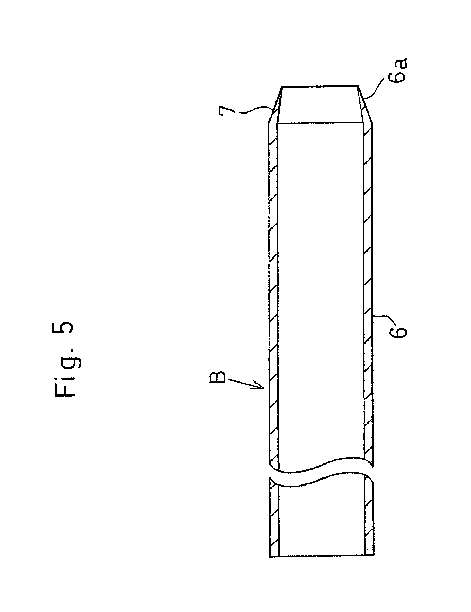

A distal portion of the introducing catheter (A) has a tubular shaft portion 5 as shown in Figs. 4 and 5. A front end 5a of the shaft portion 5 has a cone-shaped section 5e, a quasi-linear section 5d and a stepped section (Ep). The cone-shaped section 5e is tapered off (within a range of 1∼ 60 degrees, preferably 5∼ 30 degrees) toward the front end of the shaft portion 5.

A linear section 5b (0.3 ∼ 3.3 mm, preferably 1.0∼ 2.4 mm in dia.) contiguous with the quasi-linear section 5d is thinner than the quasi-linear section 5d.

The front end 5a is 5∼ 40 mm in length (L), 0.2∼ 1.5 mm in inner diameter (d) and 0.3∼ 2.0 mm in outer diameter (D). The quasi-linear section 5d measures 1∼30 mm in length (M).

From the linear section 5b toward the proximal end of the distal portion, equi-diametrical thin section 5c is formed via a taper-terminated section (En) and a stepped section (Sp).

The shaft portion 5 is formed by a synthetic resin selected from the following materials.

Those are fluoric resin (PTFE, PFE, PFA, ETFE, etc.), polyolefin (polyethylene, polypropylene, polyisobutene, etc.), polyurethane, polyamide (nylon 6, nylon 66, nylon 11, nylon 12, etc.), polyester (PET, PBT, etc.) or polyacrylate, etc.

To the above synthetic resin, added may be metallic mesh work, braided metallic wire work, glass fiber, carbon fiber, aramid fiber or the like to serve as a reinforcement.

With an outer surface of the introducing catheter (A), a hydrophilic polymer or a silicone (lubricating agent) may be coated to improve a sliding motion.

The diagnostic-therapeutic catheter (B) is formed into thin tubular configuration, and has a shorter length than the introducing catheter (A). A distal end 7 of the diagnostic-therapeutic catheter (B) is chamfered to be tapered off at its front end section 6a.

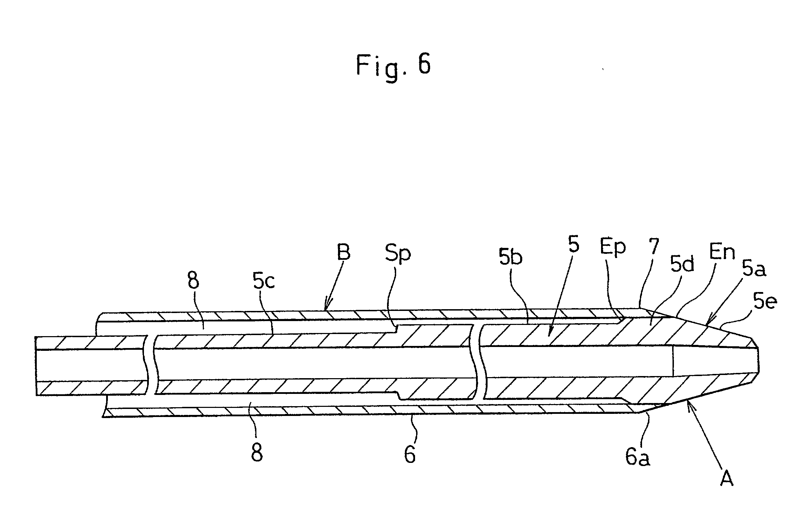

The diagnostic-therapeutic catheter (B) concentrically accommodates the shaft portion 5 of the introducing catheter (A) as shown in Fig. 6. The distal end 7 tightly engages against the taper-terminated section (En), the linear section 5b or the stepped section (Ep) without a gap (space) or clearance.

Between the diagnostic-therapeutic catheter (B) and equi-diametrical thin section 5c of the shaft portion 5, an annular space 8 is provided to supply physiological saline solution or other drugs.

With the physiological saline solution supplied to the annular space 8, a manipulator can move the introducing catheter (A) smoothly within the diagnostic-therapeutic catheter (B). The physiological saline solution expels bubbles from the diagnostic-therapeutic catheter (B) which tends to collect the bubbles due to its length extending longer.

The diagnostic-therapeutic catheter (B) is principally formed by a synthetic resin selected from the following materials.

Those are polyolefin (polyethylene, polypropylene, polyisobutene or their elastomer, etc.), polyamide (nylon 6, nylon 66, nylon 11, nylon 12 or their elastomer, etc.), polyurethane, polyester (PET, PBT or their elastomer, etc.), polyacrylate, ethylene-propylene copolymer, poly(vinyl chloride), poly(vinylidene chloride), polystyrene, poly(vinyl acetate), acrylonitrile-butadiene-styrene-butadiene copolymer (thermoplastic) or acrylonitrile-butadiene-styrene copolymer.

To the above synthetic resin, added may be a metallic mesh work, a braided metallic wire work, glass fibers, carbon fibers, aramid fibers or the like to serve as a reinforcement.

It is well known that the distal end 7 of the diagnostic catheter (B) is formed by a softer substance to avoid a human organ (e.g., heart) from being injured.

In order to minimize a resistance when inserting the diagnostic-therapeutic catheter (B) into the human organ, the distal end 7 is determined to be D25 ∼ D63 (preferably D35 ∼ D55) in terms of Shore hardness.

When the Shore hardness reduces to less than D25, the distal end 7 comes to be too soft to insert into the blood vessel. When the Shore hardness exceeds D63, the distal end 7 may come to be hard enough to injure the human organ (e.g., heart).

The materials applicable to the distal end 7 of the diagnostic-therapeutic catheter (B) are selected from the following substances.

Those are polyurethane elastomer, polyester elastomer, polyamide elastomer, polystyrene-based elastomer, polystyrene block elastomer, polybutadiene-based elastomer, hydrophilic poly(ether urethane) elastomer, hydrophilic poly(vinyl alcohol) mixture and the like.

With an outer surface of the diagnostic-therapeutic catheter (B), a hydrophilic polymer is coated to smoothly withdraw the diagnostic-therapeutic catheter (B), for example, when the diagnostic-therapeutic catheter (B) sends the spasm into the raidal artery or the brachial artery. Considering the convenience when manipulating the diagnostic-therapeutic catheter (B), the coating area of the hydrophilic polymer is in its entire length except for a near proximal end as shown at (Tu) in Fig. 3.

The hydrophilic polymer applicable to the coating is selected from the following substances.

Those are polymers having hydrophilic bases (chain bond without bridge) such as -OH, CONH₂ , -COOH, -NH ₂ , -COOH⁻ or SO ₃ ²⁻, deposited on the diagnostic-therapeutic catheter with covalent or ionic bond, i.e., carboxylmethyl starch (starch-based), carboxylmetyl cellulose (cellulose-based), alginic acid, heparin, chitin, chitosan, hyaluronate (polysaccharide), gelatin (natural soluble high polymer), poly(vinyl alcohol), poly(ethylene oxide), poly(ethylene glycol), poly(propylene glycol) ,polyacrylate, methylvinyl ether-maleic anhydride copolymer, methylvinyl ether-maleic anhydride, methylvinyl ether-maleic anhydride ammonium salt, ethyl ester-maleic anhydride copolymer, poly(hydroxyethyl phthalic ester) copolymer, poly(dimethy lolpropionic ester), polyacrylamide, polyacrylamide-based quaternary salt, poly(vinyl pyrrolidone), poly(ethylene imine), poly(ethylene sulfonate), soluble nylon or the like (aqua-soluble synthetic polymer).

When the metallic wire work or metallic pipe is built in the diagnostic-therapeutic catheter (B), its anti-kink property and torque transmission is significantly improved.

With the structure thus described, the assemble 3 is inserted into the human organ along a guide wire (not shown). Then, the introducing catheter (A) and the guide wire are withdrawn from the assemble 3 to supply the drug (e. g., contrast agent) to the diagnostic-therapeutic catheter (B). Alternatively, remedial tool (e. g., dilator) is put into the diagnostic-therapeutic catheter (B).

Such is the structure that the diagnostic-therapeutic catheter (B) accommodates the introducing catheter (A) to directly insert the diagnostic-therapeutic catheter (B) into the human organ without using the sheath.

This enables the manipulator to insert as large a diagnostic-therapeutic catheter as an 8 F size (2.67 mm in dia.) into the radial artery (V) of substantially every patient to fully treat the patient free from all anxiety.

This considerably mitigates pains the patient suffers, and at the same time, lessening the injury on the human organ tissue compared to the case in which the diagnostic-therapeutic catheter of the same size is used with the sheath.

Table 1 shows how much the assemble 3 reduces its outer diametrical dimension (OD) compared to the prior art counterpart in which has to use the sheath.

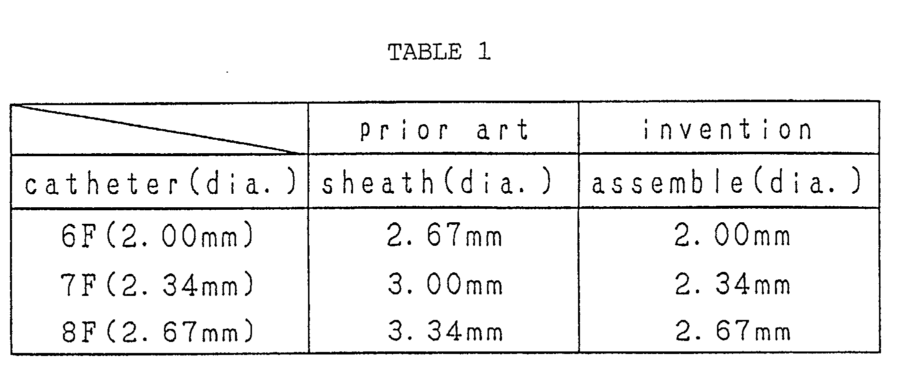

The elimination of the sheath, a sheath guide wire, a sheath dilator and a urinary catheter (used in PTCA) makes the manipulation easy, and the treatment done for a shorter period of time, while at the same time decreasing the medical cost and medical waste.

With the diagnostic-therapeutic catheter (B) and the introducing catheter (A) inserted into the blood vessel concurrently, the anti-kink property and the maneuverability of the diagnostic-therapeutic catheter (B) are improved.

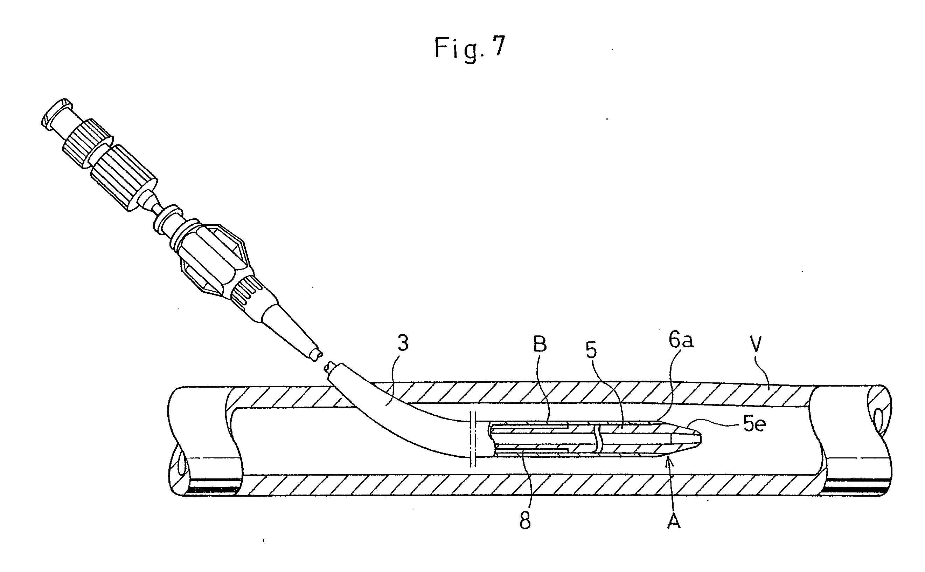

In order to treat the circulatory disease, a needle and cloak tube (not shown) are pierced into the radial artery (V) which is depicted in Fig. 7. The needle is then withdrawn from the cloak tube. After a guide wire (not shown) is inserted into the radial artery (V) through the cloak tube, the cloak tube is withdrawn.

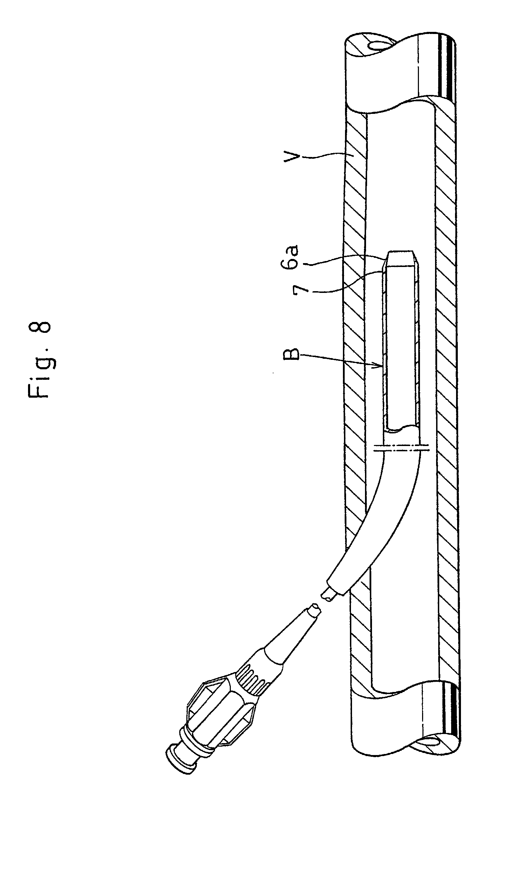

Then, the assemble 3 is inserted into the radial artery (V) along the guide wire. When the front end section 6a of the shaft portion 5 reaches the predetermined position, the introducing catheter (A) is withdrawn from the diagnostic-therapeutic catheter (B) to manipulate the diagnostic-therapeutic catheter (B) as shown in Fig. 8.

The drug (e.g., contrast agent) or the remedial tool (e.g., dilator) is supplied to the diagnostic-therapeutic catheter (B). This eliminates the necessity of the sheath to produce the diagnostic-therapeutic catheter (B) far less interventional to the patient.

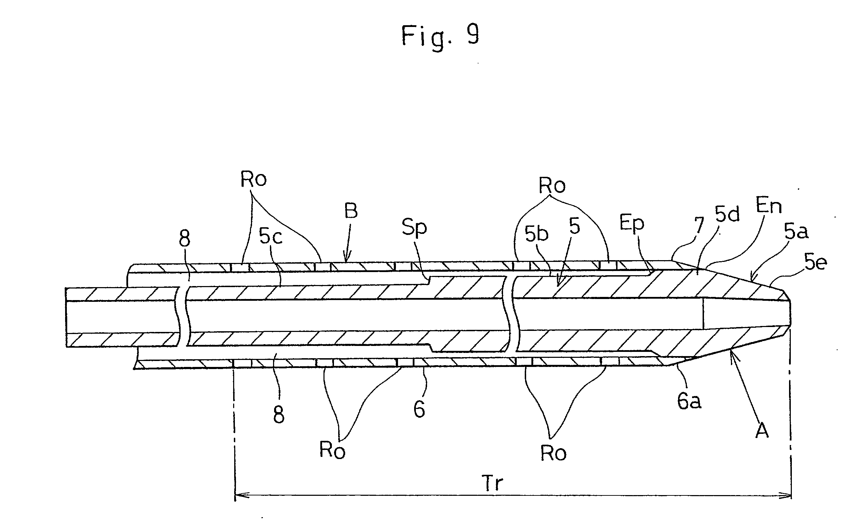

Fig. 9 shows a second embodiment of the invention in which the diagnostic-therapeutic catheter (B) has a plurality of drug-releasable side holes (Ro) extending by approx. 20 cm from the front end of the shaft portion 5 to a near proximal end portion as depicted at a distance (Tr) in Fig. 9.

The drug-releasable side holes (Ro) are in communication with the annular space 8 between the diagnostic-therapeutic catheter (B) and the introducing catheter (A). Each diameter of the drug-releasable side holes (Ro) is determined to be 1.5 mm or less (preferably 30 ∼ 100mµ) to prevent the contrast agent from inadvertently leaking out when supplying the contrast agent to the diagnostic-therapeutic catheter (B).

The anti-spasmodic drug is injected into the annular space 8 to supply it through the drug-releasable side holes (Ro) to cure the spasm which the diagnostic-therapeutic catheter (B) sent into the radial artery (V).

In this instance, the distal end 7 tightly engages with the taper-terminated section (En), the linear section 5b or the stepped section (Ep) without a space ( gap) or clearance.

This stops the anti-spasmodic drug from escaping between the distal end 7 and the shaft portion 6 so as to effectively supply the anti-spasmodic drug to the radial artery through the drug-releasable side holes (Ro).

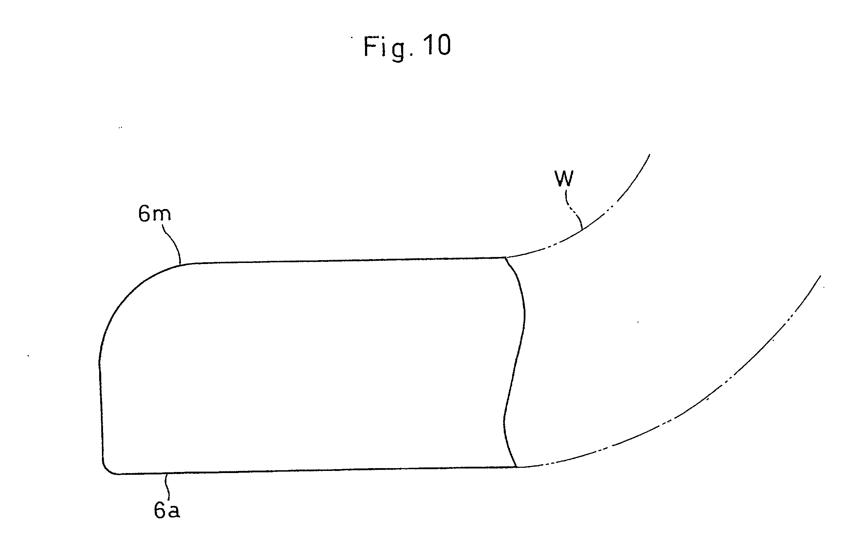

Figs. 10 through 12 show a third embodiment of the invention in which the front end section 6a of the shaft portion 5 has a bight portion (W). The bight portion (W) has a front inner edge 6m, a circumferential length of which is rounded rearward by a quarter (1/4) or less.

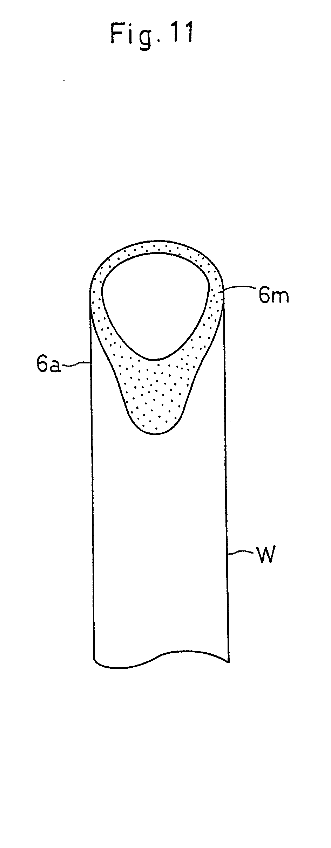

Namely, a quarter (1/4) or less of a circular cross section of the front inner edge 6m is shaved back from a distal end toward the front end section 6a as shown in Figs. 10 and 11.

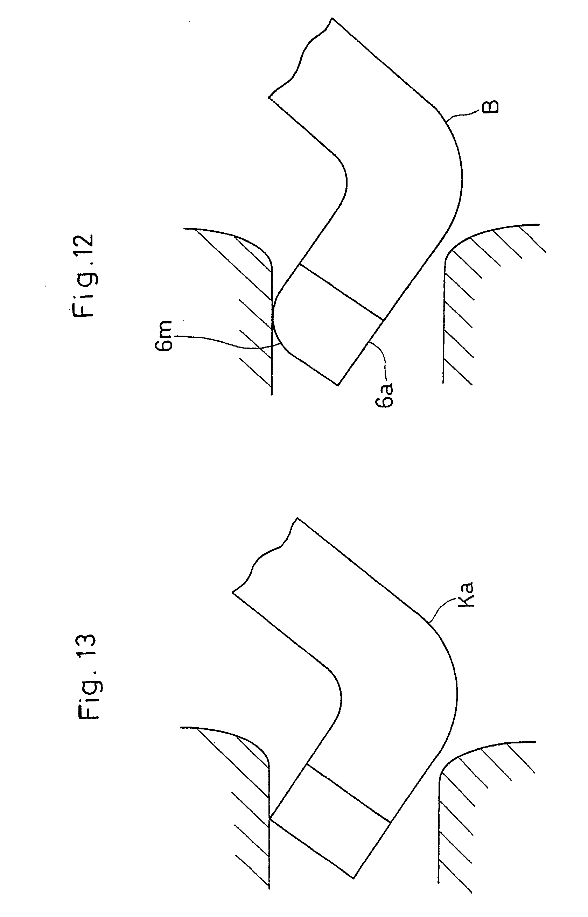

The front inner edge 6m comes to form a wider surface area (Sq) as shown in Fig. 11. The front inner edge 6m is in surface contact with the coronary artery to effectively prevent the injury against the blood vessel wall (Fig. 12) as opposed to the prior art (Fig. 13) in which a sharp edge of the catheter (Ka) encounters the entry of the coronary artery (point-to-point contact) so as to injure or dissociate the blood vessel's endothelio.

When the circumferential length of the inner edge 6m is rounded rearward by more than a quarter (e.g., 1/3, 1/2), the distal end of the diagnostic-therapeutic catheter (B) may become sharpened to injure the human organ (e.g., heart).

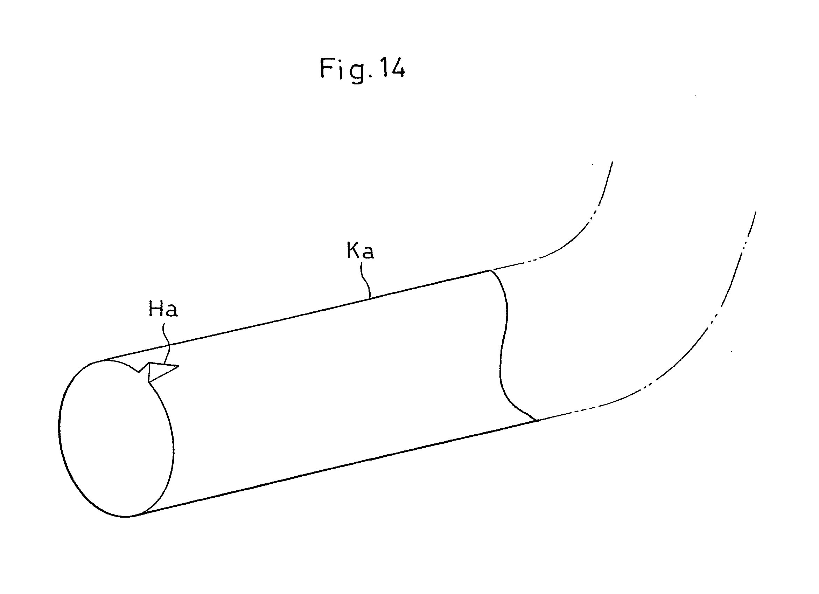

When the sheath and the catheter have a circular front end, the circular front end may injure a heart and other organs upon inserting the sheath and the catheter into the tubular human organ. Because the circular front end may locally concentrate on one point of the tubular human organ and the circular front end may be broken. The broken section (denoted by "Ha" in Fig. 14) of the circular front end may injure the human organ or the blood vessel tissue.

Because the circumferential length of the front inner edge 6m is rounded rearward by a quarter (1/4) or less, it is possible to avoid the inner edge 6m from exerting the local stress concentration against the blood vessel wall, as opposed to the prior art catheter (Ka) in which the broken section (Ha) may injure the human organ or the blood vessel tissue.

The front end of the diagnostic-therapeutic catheter (B) is formed into the cone-shaped configuration, and the circumferential length of the front inner edge 6m is rounded rearward by a quarter or less. This minimizes the resistance felt when inserting the diagnostic-therapeutic catheter (B) into the blood vessel. The rounded front inner edge 6m comes in surface contact with the coronary artery so that the coronary artery substantially remains intact.

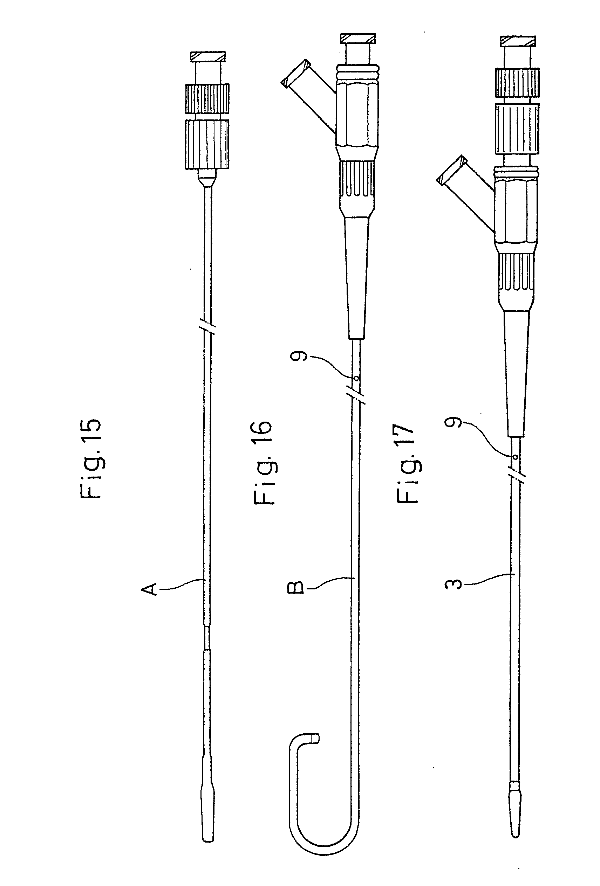

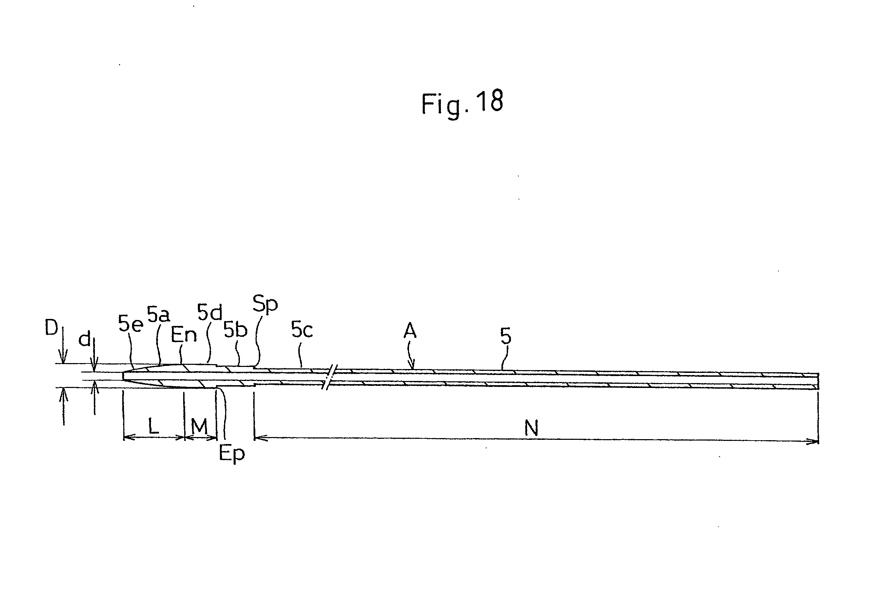

Figs. 15 through 26 show a fourth embodiment of the invention which adds like reference numerals corresponding to the component parts in the first embodiment of the invention.

The diagnostic catheter-therapeutic (B) has a drug-releasable function to provide the catheter assemble 3 of the drug-releasable diagnostic-therapeutic catheter (B) and the introducing catheter (A) as shown in Figs. 15 through 18. The diagnostic-therapeutic catheter (B) has inner and outer tubes 12, 13 which are concentrically arranged and extend along the shaft portion 5.

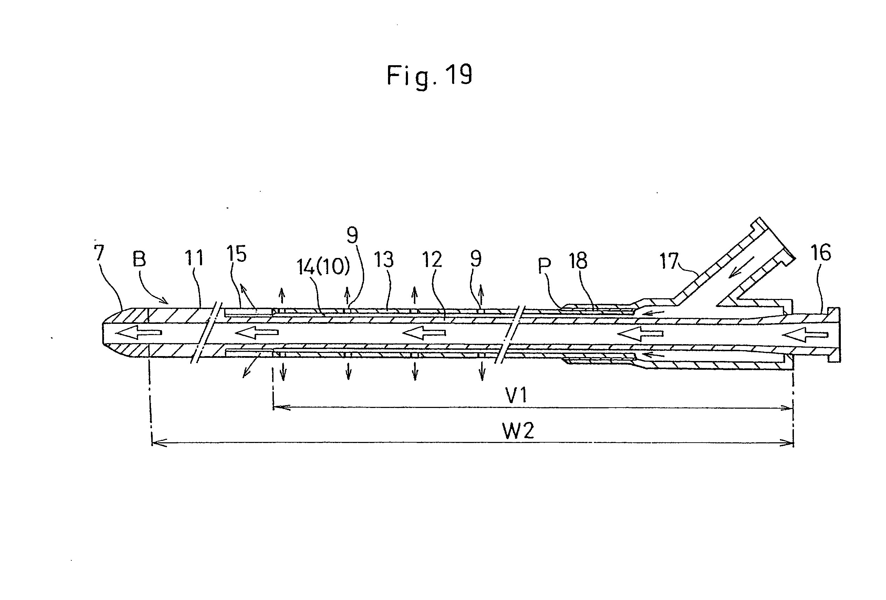

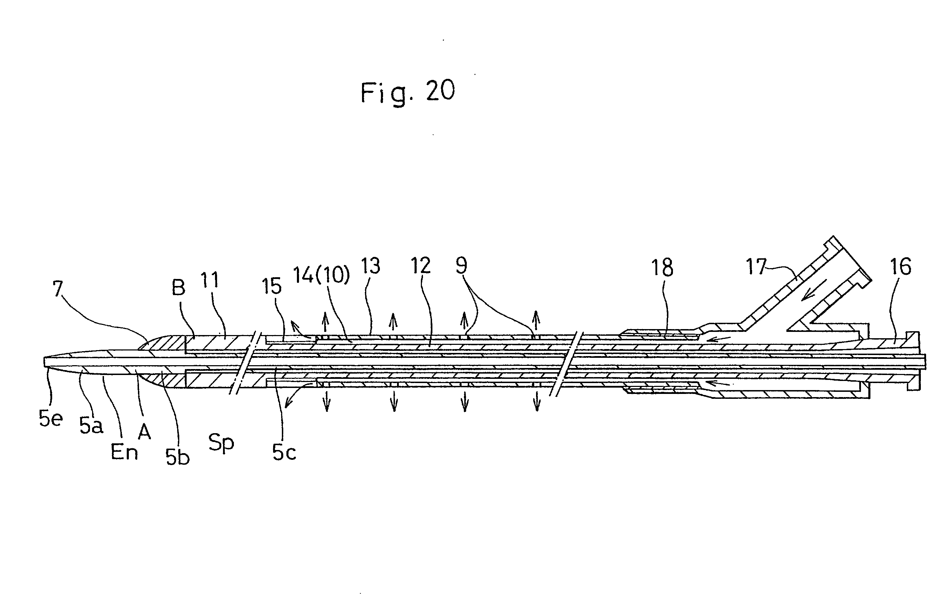

An annular space 14 is defined between the inner and outer tubes 12, 13 as shown in Figs. 19 and 20. In this situation, an open front end of the annular space 14 is entirely or partly exposed to a shaft section 11 of the diagnostic-therapeutic catheter (B) to form a drug-releasable open end 15.

It is to be observed that a braided metallic wire work can be built in. the inner and outer tubes 12, 13 along the length (W1) from the distal end 7 to a proximal end of the diagnostic-therapeutic catheter (B).

Alternatively, a metallic pipe can be built in the inner and outer tubes 12, 13 along the length (U1) from the drug-releasable open end 15 to the proximal end portion of the diagnostic-therapeutic catheter (B).

To a proximal end of the inner tube 12, is a Lure connector 16 consecutively connected to receive the drug (e. g., contrast agent).

To a proximal end side of the outer tube 13, is a Lure connector 17 connected by means of a welding or an adhesive 18 to be in communication with the annular space 14 to receive the anti-spasmodic drug (e. g., nitrile and vasolan).

The diagnostic-therapeutic catheter (B) has a plurality of drug-releasable side holes 9 (10 µm ∼ 3.0 mm in OD, preferably 30 µm ∼ 100 µm in OD) in a fashion to be in communication with the annular space 14.

By way of illustration, each of the drug-releasable side holes 9 is circular in shape, and stretched forward by approx. 20 cm from ahead of the near proximal end (P) of the outer tube 13 by approx. 30 cm.

The drug-releasable side holes 9 can be elliptic, triangular, rectangular or polygonal (slit-like) in shape.

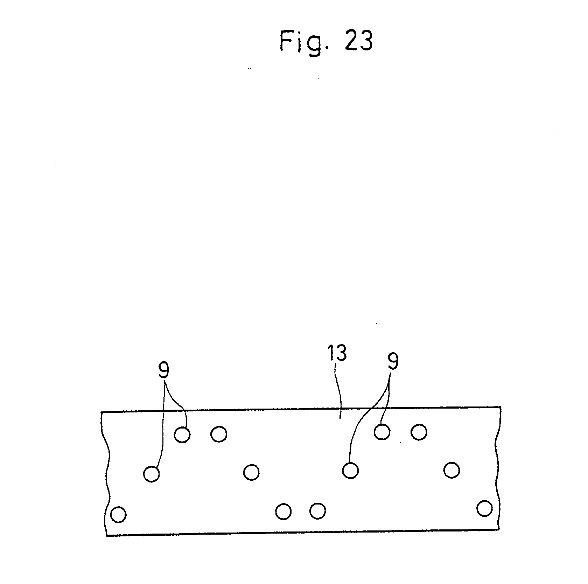

The drug-releasable side holes 9 are aligned along a helical curve as shown in Fig. 23.

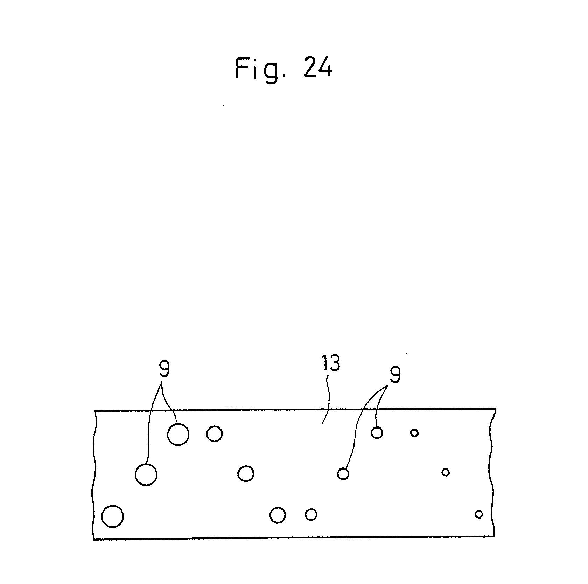

In order to adjust an amount of the drug to be released, the drug-releasable side holes 9 can be aligned so that the side holes 9 are progressively decrease diametrically from the distal end to the proximal end of the diagnostic-therapeutic catheter (B) as shown in Fig. 24.

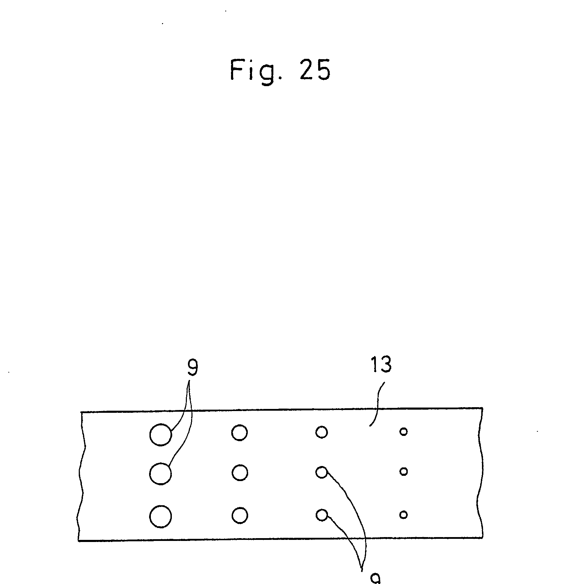

Alternatively, the drug-releasable side holes 9 can be arranged in row and column, and the side holes 9 are progressively decrease diametrically from the distal end to the proximal end of the diagnostic-therapeutic catheter (B) as shown in Fig. 25.

It is to be noted that the arrangement of the side holes 9 can be along linear, curvilinear or zigzag contours.

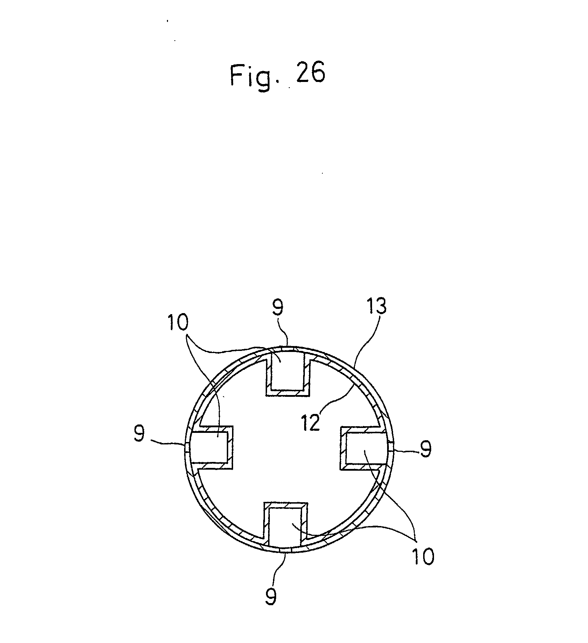

Instead of the annular space 14, one or streaks of grooves 10 are defined on an outer surface of the inner tube 12 to be in communication with the respective side holes 9 along an axial direction of the inner tube 12 as shown in Fig. 26.

The grooves 10 may be provided on an inner surface of the outer tube 13, otherwise the grooves 10 may be provided on both the outer surface of the inner tube 12 and the inner surface of the outer tube 13.

Considering a rapidity or an amount of the drug to be released, a cross section of the grooves 10 can be triangular, pentagonal, polygonal, elliptic, circular, semi-circular or hexagonal (star-like) in shape. The grooves 10 may be oriented along linear, spiral, curvilinear or zigzag contours. The presence of the grooves 10 strengthens the rigidity of the tube 12 (13) to resist more against the bending moment subjected to the tube 12 (13).

With the structure thus described, the needle and the cloak tube are cut through the radial artery (V) in the same manner as described in the first embodiment of the invention.

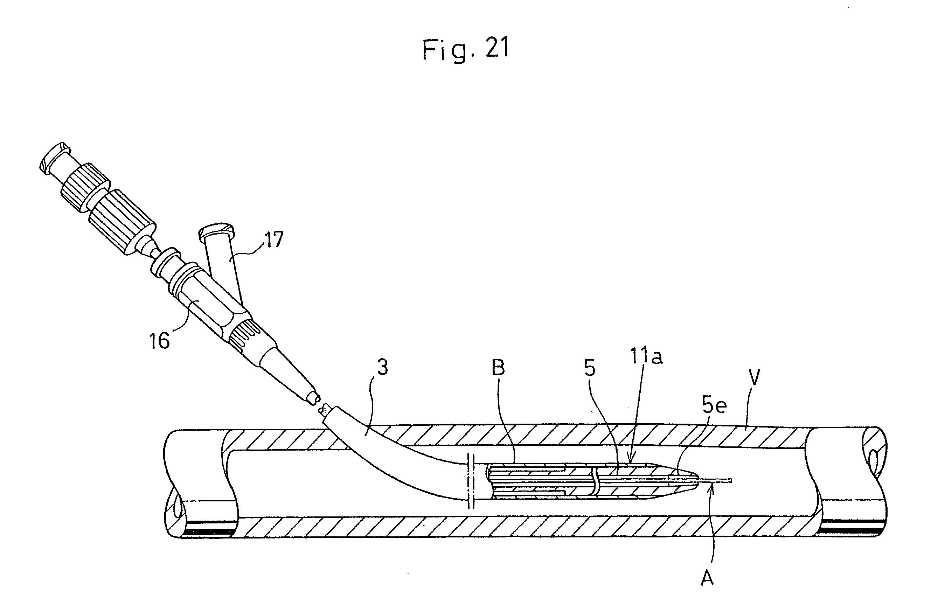

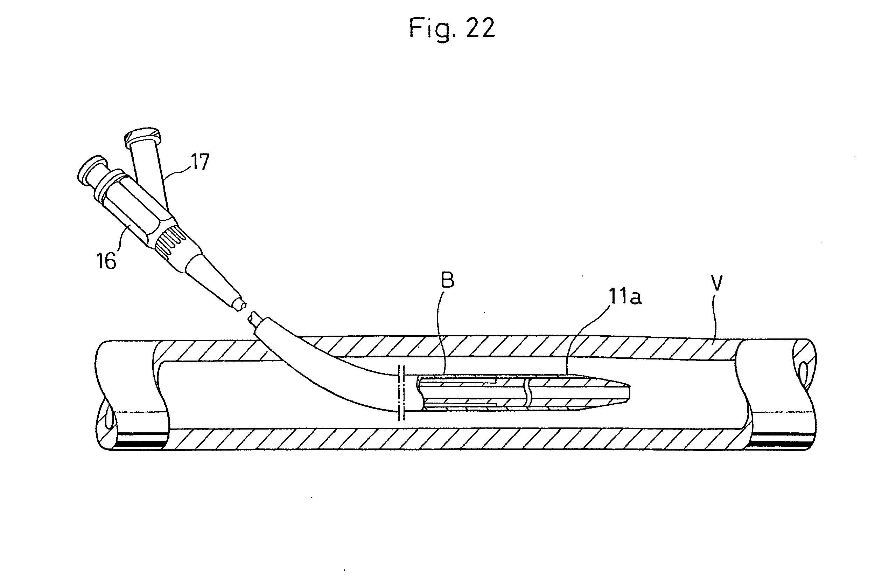

Along the guide wire in the radial artery (V), the assemble 3 is inserted into the radial artery (V) as shown in Fig. 21. Then, the introducing catheter (A) and the guide wire are withdrawn from the assemble 3 to supply the contrast agent to the diagnostic-therapeutic catheter (B) as shown in Fig. 22. Otherwise, the remedial tool (e. g ., dilator) is put into the diagnostic-therapeutic catheter (B).

Such is the structure that the diagnostic-therapeutic catheter (B) accommodates the introducing catheter (A) to directly insert the diagnostic catheter-therapeutic (B) into the radial artery (V) without using the sheath.

This enables the manipulator to insert as large a diagnostic-therapeutic catheter as the 8 F size (2.67 mm in dia.) into the radial artery (V) of substantially every patient so as to fully treat the patient free from all anxiety.

This considerably mitigates pains the patient suffers, and at the same time, lessening the injury on the human organ tissue compared to the case in which the diagnostic catheter of the same size is used with the sheath.

The structure contributes to not only eliminating the necessity of the sheath but also instantly relieving the patient of the spasm so as to render the assemble 3 less interventional to the patient.

When the spasm occurs on the blood vessel during manipulating the diagnostic-therapeutic catheter (B), the anti-spasmodic drug is injected into the Lure connector 17 to supply it through the annular space 14 (grooves 10)

The anti-spasmodic drug thus injected passes through the drug-releasable side holes 9 and the drug-releasable open end 15 to release it toward the blood vessel's endothelio of the radial artery (V) or the brachial artery so as to instantaneously relieve the patient of the spasm which the diagnostic-therapeutic catheter (B) sent into the radial artery (V) or the brachial artery.

Particularly, the drug-releasable open end 15 is effective in curing the spasm occurred on the blood vessel area (e. g., radial artery area) remote from the drug-releasable side holes 9.

With the contrast agent injected into the Lure connector 16 when a front end 11a of the shaft section 11 reaches an entry of the coronary artery, the contrast agent flows through the inner tube 12 to be released toward the entry of the coronary artery for an X-ray examination.

As evidenced by the fact that the anti-spasmodic drug passes through the annular space 14, and the contrast agent passes through the inner tube 12 individually, the passage of the anti-spasmodic drug and the passage of the contrast agent are completely separated.

This avoids the contrast agent from inadvertently leaking into an aorta through the drug-releasable side holes 9 and the drug-releasable open end 15 so as to stop the pains the patient suffers when injecting the contrast agent to release it toward the entry of the coronary artery for the X-ray examination.

With the inner tube 12 formed by the braided metallic wire work in which a thermoplastic resin pipe, metallic pipe or super-elastic metallic pipe can be built, the inner tube 12 strengthens the outer tube 13 to achieve a good maneuverability and manipulability against a curved or twisted blood vessel, while providing the tube 13 with an appropriate flexibility and anti-kink property.

Since the hydrophilic polymer is coated with the outer surface of the diagnostic-therapeutic catheter (B), it enables the manipulator to smoothly withdraw the diagnostic-therapeutic catheter (B) from the blood vessel after treating or examining the cardiovascular disease.

It is to be noted that the front end 11a of the shaft section 11 may be rounded at its inner edge by a quarter or less in the same manner as described in the first embodiment of the invention.

Upon treating the circulatory disease, the catheter assemble 3 is applicable to not merely the coronary artery and an aorta but also the blood vessel in general. The catheter assemble 3 is also applicable to the urinary catheter which involves the interior of the body.

The drug-releasable side holes 9 may be arranged so that the side holes diametrically decrease progressively from the proximal end to the distal end of the outer tube 13.

While the diagnostic-therapeutic catheter (B) has been described as hyphenated for short throughout the description, it stands for the diagnostic-and-therapeutic catheter (B).

## Claims

1. A catheter assemble comprising a diagnostic-therapeutic catheter formed into a flexible tubular configuration, and an introducing catheter which is accommodated into said diagnostic-therapeutic catheter, wherein
a distal portion of said introducing catheter having a taper-terminated portion, a linear portion, a linear-terminated portion and a stepped portion, either of which substantially engages tightly with a distal portion of said diagnostic catheter without a gap or clearance; and
an annular space provided beteween a shaft portion of said diagnostic-therapeutic catheter and said introducing catheter except for the distal portion of said shaft portion.

2. A catheter assemble according to claim 1, in which said annular space is formed between inner and outer tubes concentrically provided to extend from a proximal end portion to said distal end of said shaft portion of said diagnostic-therapeutic catheter, said annular space having a drug-releasable open end at least partially exposed to an outer surafce of said shaft portion, and said outer tube having a plurality of drug-releasable side holes.

3. A catheter assemble according to claim 2, in which said inner and outer tubes are formed by synthetic resin, and said inner tube is reinforced by a braided metallic wire work built in an area at least except for a distal portion of said inner tube.

4. A catheter assemble according to claim 2, in which at least a part of said inner and outer tubes are formed by a metallic pipe.

5. A catheter assemble according to claim 2, in which a helical, linear or curved groove is formed on either an outer surface of said inner tube or an inner surface of said outer tube, or both of them.

6. A catheter assemble according to claim 1, in which said diagnostic-therapeutic catheter has a plurality of drug-releasable side holes.

7. A catheter assemble according to claim 1, in which a distal end of said diagnostic-therapeutic catheter has a bight portion, a distal end of which has an inner edge, a quarter or less of circumferential legnth of said inner edge being rounded rearward.

8. A catheter assemble according to claim 2, in which a distal end of said diagnostic-therapeutic catheter has a bight portion, a distal end of which has an inner edge, a quarter or less of circumferential legnth of said inner edge being rounded rearward.

9. A catheter assemble according to claim 3, in which a distal end of said diagnostic-therapeutic catheter has a bight portion, a distal end of which has an inner edge, a quarter or less of circumferential legnth of said inner edge being rounded rearward.

10. A catheter assemble according to claim 4, in which a distal end of said diagnostic-therapeutic catheter has a bight portion, a distal end of which has an inner edge, a quarter or less of circumferential legnth of said inner edge being rounded rearward.

11. A catheter assemble according to claim 5, in which a distal end of said diagnostic-therapeutic catheter has a bight portion, a distal end of which has an inner edge, a quarter or less of circumferential legnth of said inner edge being rounded rearward.

12. A catheter assemble according to claim 6, in which a distal end of said diagnostic-therapeutic catheter has a bight portion, a distal end of which has an inner edge, a quarter or less of circumferential legnth of said inner edge being rounded rearward.

13. A catheter assemble according to claim 1, in which said distal portion of said diagnostic-therapeutic catheter is formed into a cone-shaped configuration in a fashion to taper off toward a distal end of said diagnostic-therapeutic catheter.

14. A catheter assemble according to claim 2, in which said distal portion of said diagnostic-therapeutic catheter is formed into a cone-shaped configuration in a fashion to taper off toward a distal end of said diagnostic-therapeutic catheter.

15. A catheter assemble according to claim 6, in which said distal portion of said diagnostic-therapeutic catheter is formed into a cone-shaped configuration in a fashion to taper off toward a distal end of said diagnostic-therapeutic catheter.

16. A catheter assemble according to claim 1, in which said distal portion of said diagnostic-therapeutic catheter is D25 ∼ D63 in terms of Shore hardness.

17. A catheter assemble according to claim 2, in which said distal portion of said diagnostic-therapeutic catheter is D25 ∼ D63 in terms of Shore hardness.

18. A catheter assemble according to claim 6, in which said distal portion of said diagnostic-therapeutic catheter is D25 ∼ D63 in terms of Shore hardness.

19. A catheter assemble according to claim 7, in which said distal portion of said diagnostic-therapeutic catheter is D25 ∼D63 in terms of Shore hardness.

20. A catheter assemble according to claim 1, in which an outer surface of said diagnostic-therapeutic catheter is coated with a hydrophilic polymer.

21. A catheter assemble according to claim 2, in which an outer surface of said diagnostic-therapeutic catheter is coated with a hydrophilic polymer.

22. A catheter assemble according to claim 6, in which an outer surface of said diagnostic-therapeutic catheter is coated with a hydrophilic polymer.

23. A catheter assemble according to claim 7, in which an outer surface of said diagnostic-therapeutic catheter is coated with a hydrophilic polymer.

24. A catheter assemble according to claim 1, in which said distal portion of said introducing catheter has a maximum diameter at its front end and a cone-shaped portion connected to said linear portion to be tapered off toward said front end, and forming a stepped portion at a linear-terminated portion, a linear area of said shaft portion having a stepped portion at a predetermined distance from said linear-terminated portion, said distal portion further having a diameter-reduced lean portion from said stepped portion to a proximal end with an equi-diameter thickness through an entire length of said proximal end.

25. A catheter assemble according to claim 2, in which said disatal portion of said introducing catheter has a maximum diameter at its front end and a cone-shaped portion connected to said linear portion to be tapered off toward said front end, and forming a stepped portion at a linear-terminated portion, a linear area of said shaft portion having a stepped portion at a predetermined distance from said linear-terminated portion, said distal portion further having a diameter-reduced lean portion from said stepped portion to a proximal end with an equi-diameter thickness through an entire length of said proximal end.

26. A catheter assemble according to claim 6, in which said distal end portion of said introducing catheter has a maximum diameter at its front end and a cone-shaped portion connected to said linear portion to be tapered off toward said front end, and forming a stepped portion at a linear-terminated portion, a linear area of said shaft portion having a stepped portion at a predetermined distance from said linear-terminated portion, said distal portion further having a diameter-reduced lean portion from said stepped portion to a proximal end with an equi-diameter thickness through an entire length of said proximal end.
